# EUROPEAN PATENT SPECIFICATION

(11) **EP 1 689 882 B1**
(45) Date of publication and mention of the grant of the patent: **26.11.2014**
(21) Application number: 04775740.6
(22) Date of filing: 28.01.2004
(51) Int. Cl.: G01N 33/53, G01N 33/60, C12Q 1/68, C07H 21/02, C07H 21/04, C07K 14/00

(54) **RADIOACTIVE MULTIPLEXING ANALYTICAL METHODS**
RADIOAKTIVE MULTIPLEX-ANALYSEVERFAHREN
PROCEDE D'ANALYSE AVEC MULTIPLEXAGE DE RADIOACTIVITES

(30) Priority: 07.10.2003 US 680277
(43) Date of publication of application: 16.08.2006
(73) Proprietor: TRAN, Nathaniel Tue, Temecula, CA 92590 (US)
(72) Inventor: TRAN, Nathaniel Tue, Temecula, CA 92590 (US)
(74) Representative: Hart, Deborah Mary
(86) International application number: PCT/US2004/002442
(87) International publication number: WO 2005/045056

(56) References cited:
- EP-A- 1 158 057
- WO-A1-02/059365
- WO-A2-00/63701
- WO-A2-00/63701
- US-A- 4 016 250
- US-A- 4 524 275
- US-A- 5 807 522
- US-A1- 2002 006 623
- US-B1- 6 203 993
- US-B1- 6 203 993

## Description

### FIELD OF THE INVENTION

This invention related to laboratory analytical methods, specifically the methods here allows both samples to be compared to be co-analyzed simultaneously for more reliable results. The invention has applications in proteomics, phosho-proteomics, glycomics, metabolomics, transcriptomics and genomics analyses.

### BACKGROUND OF THE INVENTION

Many analytical methods require side-by-side or sequential comparison to quantitatively compare differences between two samples. For example, to compare proteins on a Western blot, samples are run on adjacent lanes; to compare small molecules on HPLC, samples are run sequentially one after the others. This format of analysis requires twice the effort in sample preparation; in addition, variability can be introduced into the system if the treatments of one sample slightly deviated from another. For these reasons, many repetitions plus statistical analysis are required before any differences become credible. A method that allows two samples to be mixed together for analysis and quantitative comparison will provide significant advantages in analytical precision.

### Existing methods:

Multiplexing methodologies have been around for a while especially the use of different color dyes for comparative labeling. For example, a tissue section can be used for simultaneous multiple immuno-histochemistry experiment by using antibody conjugated to different color dyes. The intensity of each dye is used to compare the relative abundance of each type of antigens that the different color-coded antibodies bind to.

Recently fluorescent dyes of different colors are used to label DNA, RNA, and even proteins to enable two or more of these samples to be mixed together and analyzed simultaneously. One application is a DNA array where DNA or RNA from different samples are labeled with dyes that fluoresce at two different wavelength, mixed, applied to the same array for competitive binding and then read to see which sample has more of which genes expressed. Another important application is the labeling of two proteins samples by different fluorescent colors dyes such as Cydyes® sold by Amersham Biosciences and Alexa® fluor dyes sold by Molecular Probes. These protein samples are then mixed and co-separated by 2-dimensional gel electrophoresis into thousands of dots based on the proteins' differences in isoelectric points and apparent molecular weights. A fluorescent scanner is used to read Cy2®, Cy3® or Cy5® signal separately and computer software compare the signal intensity between them for quantitative comparison of protein abundance between test samples.

### Labeling and detection

The current labeling techniques for fluorescent labeling require coupling of bulky and disruptive fluorochromes to amino acids or nucleotides within the molecule of interest to facilitate detection. While DNA and RNA often can accommodate structural modifications caused by covalent linkage with dyes for DNA array analysis, proteins cannot for protein array analysis. A fluorochrome such as Cydye® often will modify amino acids, such as lysine, and will effectively change the epitope structure that the lysine is involved in. As a result, the label interferes with the analyte and often prevents an antibody from binding normally to labeled proteins the same way it would to unlabeled proteins. Furthermore, dye may alter the structure of DNA or RNA significantly, such that proteins, e.g., transcription factors, won't be able to recognize and bind as they would do with native sequences.

Radioactive labeling solves the problem by making the labeling group much smaller, or better yet, by isotope replacement with identical atoms. Additionally, radioactive labeling is at least 100 times more sensitive than many other methods upon detection. Replacement labeling can be done with direct incorporation of labeled precursors such as amino acids yielding labeled proteins with absolutely no chemical modification. Such metabolic labeling techniques are done routinely in research laboratories. Once labeled, X-ray photography can be used to detect and record the respective radiation. Since X-ray film has a limited linear range thus usually not used for quantitative analysis, other methods have evolved over time. A commonly used method comprises the use of a phosphorescent storage imaging screen. The high-energy radiation excites the storage material's electrons into their phosphoresced state at which they will remain until excited again by the right quanta of energy. Using that principle, a phosphorescent screen captures some of the radiation energy, stores it, and gives back when read with a tuned laser. Such devices are also commonly used in photography. Another method that enables quantitative analysis of radiation is Scintillation Counting. A scintillation material is mixed with the radioactive sample so that when the material is struck with high-energy radiation it will give up light for easy detection and quantification. Direct detection with devices such as Geiger counter is also possible; however, the cost for making such instruments has limited its use.

WO 02/059365 describes a method and apparatus for simultaneous quantification of the amounts of one or more radio active nuclides within arbitrary regions on a surface where these nuclides have been deposited, adsorbed or fixed.

US 6,203,993 relates to methods useful for identifying and analysing nucleic acids, especially variants of single nuclotide polymorphisms, that are indicative of disease or the predisposition for disease.

### SUMMARY OF THE INVENTION

In its broadest sense, the invention concerns subject-matter as defined in the appended claims.

In detail, the present invention discloses a method for comparing samples comprising the steps of: a) providing two samples for comparative analysis; b) labeling molecules in a first sample by covalent linkage with a first radioactive isotope; c) labeling molecules in a second sample by covalent linkage with a second radioactive isotope which has a different half-life compared to the half-life of said first radioactive isotope; d) mixing said first sample and said second sample into a homogeneous mixture; e) subjecting said homogeneous mixture to any means of separation that can separate the molecules into fractions or bands or dots; f) quantifying a total amount of radiation from each fraction or band or dot; g) quantifying a total amount of post-decay radiation from said each fraction or band or dot after a period of radioactive decay; h) examining every fractions or band's or dot's total radiation and total post-decay radiation whereby any fraction or band or dot with a deviated ratio of total radiation to total post-decay radiation can be identified. A differentially abundant molecule from said fraction or said band or said dot with deviated radioactive isotopes' ratio can be isolated wherein said differentially abundant molecule is responsible for causing said deviated radioactive isotopes' ratio. The reading results can be used to make quantitative comparison of similar molecules between the tested samples. Alternatively, if one sample has a known amount of one or more molecules, then the comparison allows quantification of these molecules in the unknown tested sample.

This invention provides a novel method for coding individual samples, enabling the samples to be homogenized, testing the samples simultaneously and precisely the same way, then distinguishing and quantifying which molecules belong to which samples. One innovation is in the method that enables quantification of more than one isotope in a mixture. This is accomplished by exploiting the difference in their half-life. By quantifying total radiation, then storing the sample for radioactive decay (while preserving other attributes) before quantifying total radiation again, the amount of radiation between isotopes with different half-life can be selectively quantified. In addition to the time and cost savings by running two or more samples simultaneously, the increased reliability also provides new possibilities for analysis.

The main object of this invention is to provide an improved method for biomarker discovery. Differentially abundance molecules between two samples can be isolated for further studies. Generally two samples, whether drug treated compared against vehicle treated, or diseased compared against healthy, are labeled, mixed together, and separated by any number of means commonly known to one skilled in the art, into many fractions, bands, or dots. For example, molecules may be separated by chromatography, electrophoresis, immunoprecipitation, immunomagnetic capturing, array profiling, differential extraction or precipitation such as salt-cut precipitation or organic solvent extraction or precipitation, heat-treatment precipitation, microfluidic device, capillary electrophoresis, differential centrifugation, or gradient separation...etc.

The ratio of radioactive isotopes in each fraction can be easily monitored. Any fractions whose ratios deviate from a standard ratio can be examined further to identify the exact molecules in those fractions that are responsible. These are the molecules of interest because their levels of abundance vary between the tested samples. The molecules can be identified by mass spectrometry and used as biomarkers for drug efficacy or diseased condition. Radioactive labeling with different isotopes also results in same molecules of different mass that can be differentiated and quantitatively compared during mass spectrometry analysis.

Another object of this invention is to provide a method for studying different degree of modifications to a particular type of molecules such as post-translational modifications of proteins, or methylation of DNA. One set of sample is labeled with ³²P phosphates while the other set undergoing a different treatment is labeled with ³³P phosphates to study differential phosphorylation. Also described is that one set of sample is labeled with ³H sugar such as mannose while the other set is labeled with ¹⁴C mannose for differential glycosylation studies. Phosphorylation or glycosylation samples can also be labeled with another isotope such as ³⁵S to determine the proportion of proteins that are modified. The two sets are mixed for analysis after labeling allows an unsurpassed degree of comparison. Other forms of post-translational modifications such as methylation, farnesylation, ubiquitination...etc. can also be studied by varying the types of labels use. These variations will be apparent to those skilled in the art.

Also described is the application of the technique toward comparing DNA methylation throughout the genome. One set of DNA can be methylated with ³H donor groups while the other set methylated with ¹⁴C donor groups. The DNA is then mixed together, digested into smaller fragments, and profiled onto a DNA array. Aberrations in signal ratios between the isotopes signify variations of the degree of methylation for particular captured genes.

A further object of this invention is to provide an improved method for genomic and proteomic analysis using radioactive isotopes of different half-life to enable simultaneous processing and quantification of multiple samples of DNA, RNA, proteins, and other molecules without damaging or rendering these molecules incomparable or incompatible for analysis purposes, e.g., chemically modifying them. Additionally and importantly, radioactive labeling also provides much higher sensitivity than any other methods of labeling.

### BRIEF DESCRIPTION OF THE DRAWINGS

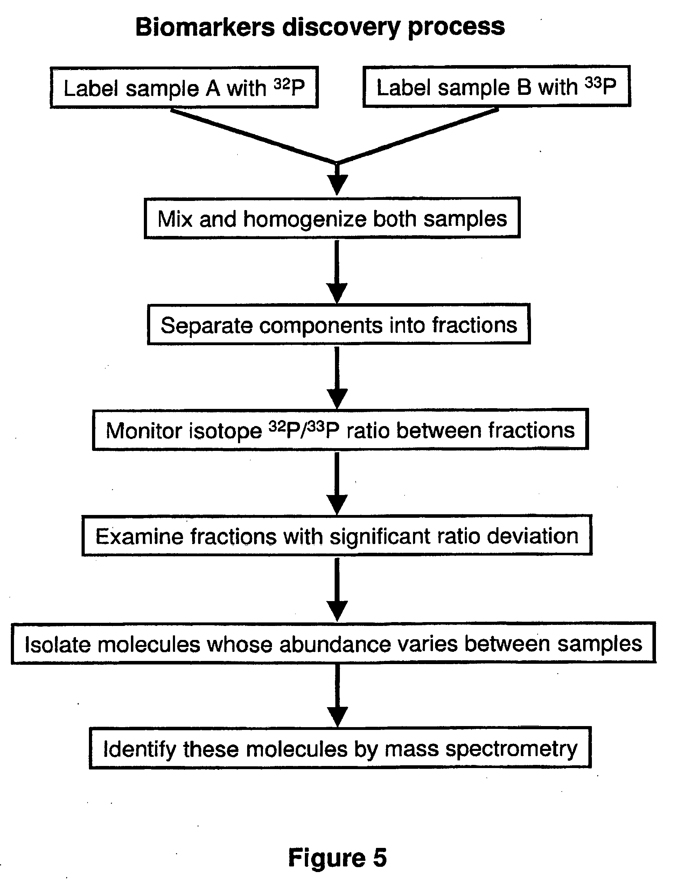

Figure 1 and 5 is a flow-chart describing the biomarkers discovery process using different radioactive isotopes to encode molecules from two samples and the isotopes' differences in radiation energy to decode them after analysis (for comparison reasons).
Figure 2 is a flowchart describing a biomarker discovering process using different radioactive isotopes to encode molecules from two samples and the isotopes' differences in half-life to decode them after analysis.

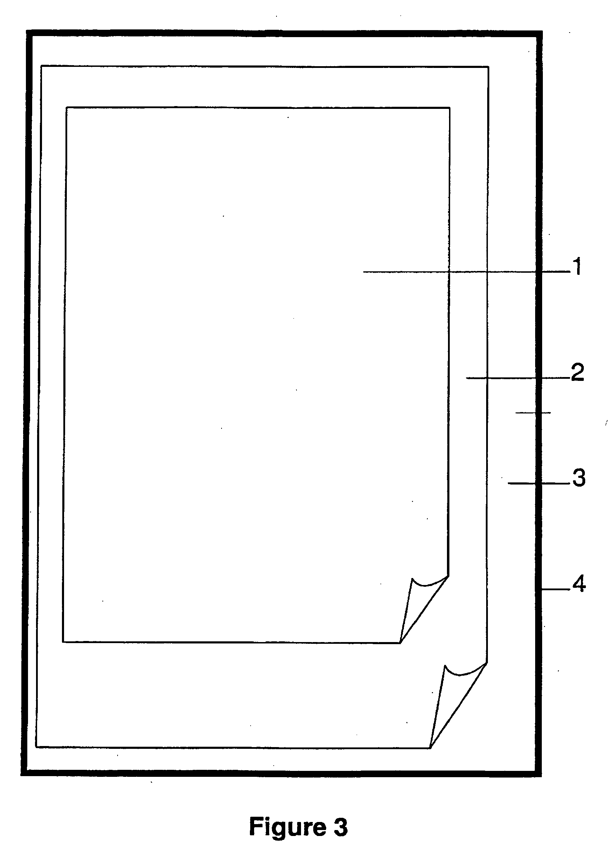

Figure 3 is front view of a multi-screen phosphorescent storage and imaging system; and

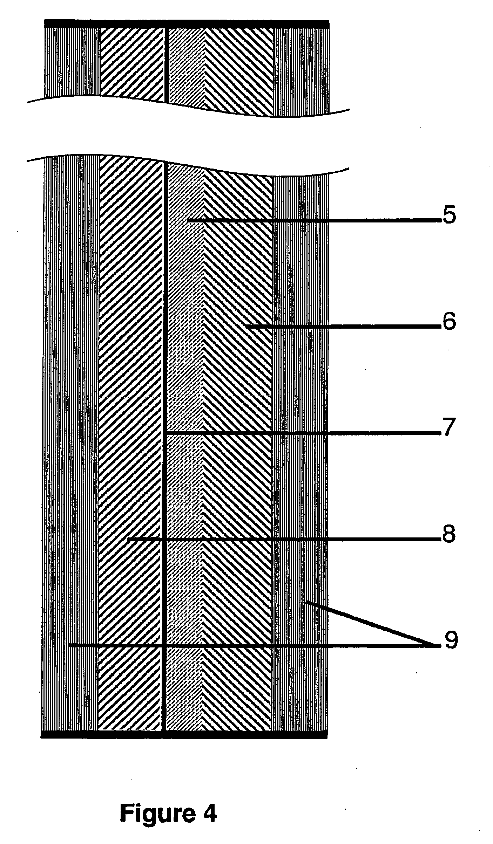

Figure 4 is a cross-section side view of an array that enables easy detection and quantitative comparison between multiple isotopes.

### DETAILED DESCRIPTION OF THE INVENTION

The invention provides a labeling and detection method for comparing profiles of a plurality of molecules derived from comparable sources. The method employs the use of radioactive isotopes which offer superior sensitivity for detection of any existing methods. In addition, radioactive isotopes can be incorporated directly into the molecules of interest eliminating any need for making any chemical modification to these molecules. To support the method, the invention also teaches ways to make supporting devices for increase performance of the method.

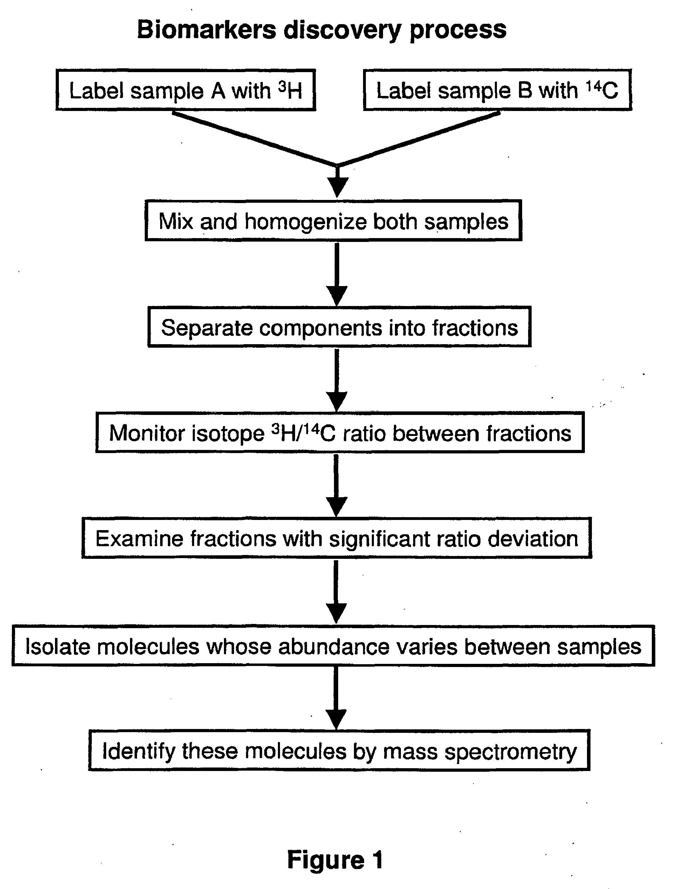

With reference to Figure 1, described is a method of discovering biomarkers and developing high-throughput assays using these biomarkers. Samples from treated and untreated cells labeled with different isotopes such as ³H or ¹⁴C are mixed together for analysis. The mixture is subjected to different types of chromatography one after another hereby refers to as tandem chromatography. The chromatography fractionates proteins based on their variations in size, charge, affinity to certain groups etc... The fractions are continuously monitored to find any fraction with deviation in ³H/¹⁴C isotope ratio. The fractions with significant ratio deviation are further analyzed to single out the exact molecules responsible for the variation. These molecules are then identified by mass spectrometry. Then monoclonal antibodies against these molecules are made as component for an array for high-throughput screening. The array is used to capture and compare proteins from the original samples to validate the changes and the array itself. Then the array is ready to be used to screen for similar changes. For example, a working drug with known therapeutic efficacy can be used to produce biomarkers in a simple system such as a cell culture model. The discovered biomarkers are then used to make antibody arrays for high-throughput screening of other potential compounds with the same cell culture model.

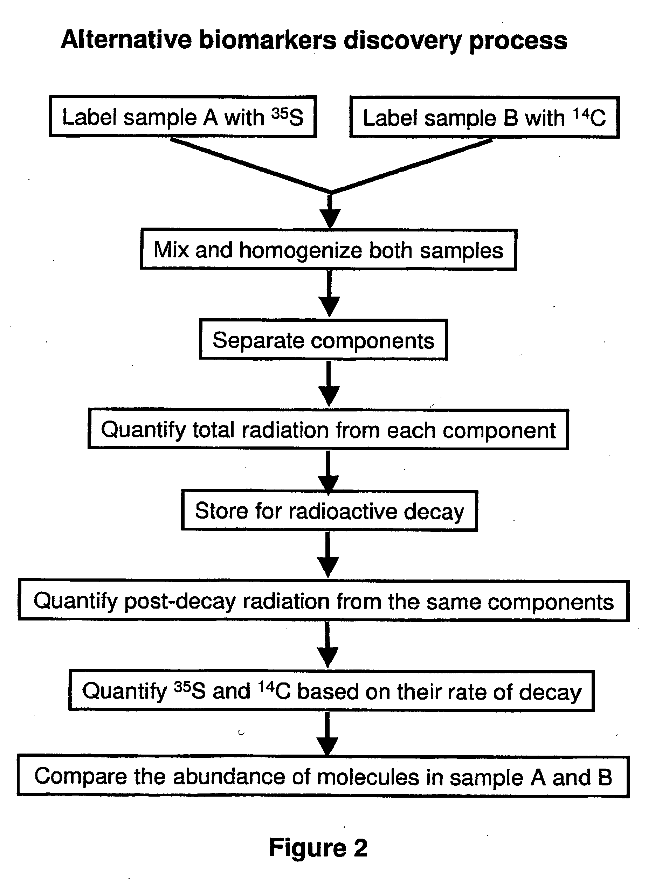

With reference to Figure 2, illustrating the present invention, mixtures containing at least two isotopes having significant differences in half-life are used for the purpose of quantitative analysis and comparison. This method of analysis can also be performed where the selective blocking method fails. An exemplary example is ¹⁴C (156keVmax) and ³⁵S (167keVmax). When ¹⁴C and ³⁵S are used together; a suitable method of analysis is used to quantify total radiation, then store for a decay period and then quantify total radiation again. The pre-decay and post-decay amounts of radiation read are used to calculate the amount unique to each isotope. This is possible because these two isotopes have different half-life: 5730 years for ¹⁴C and 87.4 days for ³⁵S. For simplicity, let's say the time period between the first quantification and the second quantification is 87.4 days which is exactly equal to one half-life of ³⁵S. Then the signal difference between the first read and the second read must be equal to half the total ³⁵S signal from the first read. As a result signal unique to ³⁵S or ¹⁴C can be calculated.

In the present invention, at least two samples can be analyzed and compared by labeling with radioactive isotopes of different half-lives. The labeling may be done by covalent linkage like the one using NHS ester or by incorporation if the samples comprise live cells and are actively synthesizing the molecules of interest. Once labeling is completed, the two samples are mixed for analysis. For nucleic acids samples, the analysis can include but are not limited to gel electrophoresis, chromatography, and DNA array. For protein samples, analyses like protein array, SDS-PAGE, Immunoprecipitation, 2-d gel for proteomic analysis, various forms of chromatography including HPLC are preferred choices.

In another preferred embodiment, the labeling of molecules with different radioactive isotopes is used to quantitatively compare subcomponents of molecules. These comprise certain characteristic of molecules such as the degree of phosphorylation of proteins or the degree of methylation of DNA. The degree of existing phosphorylation of proteins from two samples can be compared by further phosphorylating these proteins with ³²P or ³³P phosphates. The amount of labeled phosphates quantified on these proteins is thus indicative of how many phosphorylation sites are available. Such availability is indicative of preexisting degree of phosphorylation. Similarly, DNA methylation from two samples can be compared.

The above methods can also be combined to quantify isotopes' signal from a mixture of ³H,¹⁴C and ³⁵S. The total radiation is first read and then the partial radiation passing through a screen is then read. The screen is designed to block proportionally higher percentage of radiation from tritium, thus tritium signal can be calculated from the first two read (for comparison reasons). Then the mixture, gel or array is stored for a significant amount of time relative to ³⁵S's half-life. Then the sample is read again for total radiation. The reduction in signal is mostly due to ³⁵S because even for 87 days, tritium with a half-life of ∼12 years and ¹⁴C with a half-life of ∼5730 years hardly decay at all. To improve reliability, dots of just ³H, ¹⁴C, and ³⁵S should be added to the gel or array as internal standards to base the calculation on.

The use of specialized software can significantly automate the task minimizing operator errors. The user simply need to specify the standard dots representing single isotope of ³H, ¹⁴C, or ³⁵S from all three exposures and the computer can do the rest. Even if exposure time may be inconsistent between the three times, there is enough data to perform the calculation to obtain the required signal reading for individual isotope. Alternatively, when standard dots are not available, the software can ask for exposure durations and decay time to perform the calculations necessary to obtain signal for individual isotope.

The most useful applications for this technique are for biomarkers discovery. Proteins can incorporate ³H and ¹⁴C labeled amino acids or even ³⁵S when Cystein and Methionine are used for double-label or triple-label multiplexing analysis. In addition, multiple labels can be used for studying protein modifications, and interactions. Glycosylation can be differentially labeled with ³H or ¹⁴C glycan while proteins are labeled with ³⁵S. Phosphorylation can also be compared with ³²P and ³³P while protein abundance is compared with ³H and ¹⁴C (for comparison reasons). The quadruple labeling also serves to determine which proportion of proteins is phosphorylated as well as comparing relative amount of phosphorylation and relative amount of protein expression all in one experiment.

Currently, radioactive Cystein and Methionine are only available commercially with ³⁵S label while other amino acids are available with both tritium and ¹⁴C labels. The main reason is because there is little value for using tritium or ¹⁴C labeled Cystein or Methionine; as a result, these amino acids are not made and sold commercially. With the introduction of radioactive multiplexing techniques, these amino acids will become much more useful especially because they enable triple labeling when ³H, ¹⁴C, and ³⁵S Cystein and Methionine are used for the experiment. Besides the triple labeling experiment, many enzymes can be severely affected when normal hydrogen is replaced with tritium. Because tritium weight three times more than hydrogen, tritium molecular kinetic is much lower. An enzyme reaction that normally involves the exchange of a hydrogen atom will become significantly slower when tritium replaces the hydrogen. As a result, such replacement can significantly alter cellular metabolism, an event that is highly undesirable due to the nature of the experiment. To overcome such shortfall, ¹⁴C labeling can be used to compare with ³⁵S labeling.

In addition to the amino acids themselves, their metabolic precursors can also be used to perform the same labeling experiment. These metabolic precursors are molecules that can be easily converted and incorporated into protein as Cystein or Methionine while retaining the radioactive label. For labeling to be effective, such precursor encompassed molecules that are unique to Cystein or Methionine biosynthesis pathways, and derivatives of post synthesis. A good example is Cystine, the oxidized form of two Cysteins covalently linked by a sulfide bond. Other slightly modified versions of Cystein or Methionine or larger molecules containing these amino acids as components can also be used as precursors. These precursors are known to those skilled in the art and can vary slightly depending on the biological system used. In addition, when this method becomes more useful, those skilled in the art will be able to apply atomic replacements of nitrogen and oxygen with other radioactive atoms to enable even more multiplexing capability.

### Specific methods of analysis:

### Array-

Protein array is a method that will benefit greatly from this novel approach. An array is used for profiling and analyzing many molecules simultaneously using just minute amount of sample. The current method for detection and quantification of proteins or DNA/RNA on the array use fluorescent dyes, thus the array support only needs to be a sturdy structure that does not fluoresce. Described is an array's support that also serves the dual purpose of a screen, which blocks higher proportion of radiation from one particular label but not the others. Depending on the thickness and type of material used, such screen can allow differentially detection and quantification of tritium, carbon-14, sulfur-35, phosphorus-33, phosphorus-32, and other radioactive isotopes from each others. The screening technique is particularly useful for radioactive labeled samples whereby at least most of signal from one label is blocked by the screen while higher percentage of the other signal is allowed to pass making simultaneous detection and quantification of both signals possible.

Another variation of the array support described is the inclusion of scintillation material to convert radiation into light for easier detection. The array can have a sturdy transparent supporting material such as glass, then a layer of scintillation, then a thin film covering material. The thin film will also serve the dual purpose of a blocking screen which will be extremely useful for selectively blocking tritium while allowing a high percentage of ¹⁴C or ³⁵S signals to pass. On top of the thin film is the activated surface where the antibodies are spotted for capturing and profiling antigens. Once captured, the unscreened radiation from these antigens can be read by placing a second scintillation layer on top of the array and then reading the light emitting as a result of radiation striking scintillation materials. The partially screened radiation can also be read simultaneously from the bottom of the array by reading the light from the embedded scintillation layer. Confocal optical technology allows high resolution reading that can essentially bypass the thickness of the glass layer. To avoid signal crossing over between the top and the bottom screen, the thin film acting as blocking screen also block emitting light. Thicker or denser variation of this screen can also be used to differentially block ³²P and ³³P for DNA arrays or phosphorylation studies.

In yet another variation described, a thin film of phosphorescent material is used in place of the first and second scintillation material layers described above. The phosphorescent material can capture and store the radiation signature to be read back with a properly tuned laser. To read the array, the phosphorescent material is erased by photo-bleaching and then the array is also exposed to another phosphorescent screen "cover slip" on top of the array for equal amount of time before both screens are read.

DNA arrays will also benefit where higher detection sensitivity is needed. Low abundance DNA or RNA can be detected more easily. Even direct detection is possible eliminating the extra amplification steps such as PCR. Other detections for things that cannot be amplified such as DNA modification are also possible. In addition, pre-purification or other enrichment methods can be used prior to array analysis. These methods can range from eliminating certain high abundant DNA/RNA to specifically trapping certain types of DNA/RNA.

### Tandem/multidimensional chromatography for proteomic analysis

Chromatography methods have not been able to replaced 2-D gel electrophoresis probably due to the lack of a multiplexing approach. Unlike electrophoresis where sample can be compared side by side or multiplexed on the same gel, chromatography of two samples usually have to be done sequentially for comparison. One type of chromatography can separate samples into fractions which can later be used to separate further by another type of chromatography. It is unlikely that the same sample run back to back on many sequential types of chromatography would yield exactly the same set of fractions. Therefore, the method has not been popular for comparing samples. However, with our labeling and detection method, two samples can be mixed together to be co-separated by sequential chromatography. Since the molecules in both samples possess the identical structure and chemistries (from metabolic labeling or covalent linkage with chemically similar tags), the molecules are separated equally regardless of which sample they come from. A radiation detector can monitor the proportion of the isotopes in real-time through a flow cell or small aliquots from fractions can be removed and counted by scintillation counting. Samples labeled with ³H and ¹⁴C, or ³²P and ³³P can readily be distinguished by existing scintillation counter (for comparison reasons). Radiation of aliquots from the same fraction can be determined before and after a decay period to allow differential quantification especially for ¹⁴C and ³⁵S.

### Immunoprecipitation:

This is also one of the methods of analysis that will benefit greatly with the multiplexing technique. Current immunoprecipitation experiments involve multiple samples treated in similar manners in parallel. Many sequential steps such as incubating with samples and washing can introduce variation. Sometimes, the amounts of capturing agents recovered also vary because some are inadvertently washed away. Having multiple samples in one run ensure that these samples are always treated equally enabling true comparison.

With the increase in reliability, there can also be other methods similar to immunoprecipitation used to compare any particular molecules between samples. For example, an immobilized DNA sequence can be used to capture labeled RNA from two samples for comparison. Then optionally the captured molecules can be separated later for further analysis if necessary. An obvious advantage of this method is to replace the labor intensive Northern blotting method.

### Labeling:

In addition to metabolic incorporation of amino acids, nucleotides or other precursors that are made with one or more atoms replaced with radioactive isotopes, other bioconjugation and molecular replacement methods can also be used. A well-known method is iodination using ¹²⁵I to modify Tyrosines. Other coupling methods that add a group of molecules can also be used. These methods use NHS ester, cyanogen bromide, bis-oxirane (epoxides), carbonyldiimidazole, sulfonyl chloride, periodate, fluoromethyl pyridinium sulfonate, glutaraldehyde, acetaldehyde, and formaldehyde...etc to create covalent linkage.

A gentle way to label protein with at least one phosphorylation site is to use a kinase to add a phosphate group with ³²P or ³³P. Protein with known phosphorylation sites can be phosphorylated with specific kinase. The reaction can be used to label protein or to determine the amount of existing phosphorylation in these proteins by quantifying the amount of available phosphorylation sites.

### Methods of detection:

Radiation detection and quantification is made possible by exposing to phosphor-imaging screen which store radiation energy to be read back. Another way to quantify radiation is to use scintillation materials to convert radiation into lights and then detect and quantify the amount of generated light. Using electronic detectors that can detect radiation directly is also possible, but is more costly.

Mixture of isotopes such as ¹⁴C and ³⁵S, or ³²P and ³³P can be quantified by reading radiation from one set of aliquots first, while storing a duplicate set of aliquots for decaying to be read later on. The pre-decay and post-decay amounts of radiation are used in combination with the isotopes' half-life to determine the amount of radiation unique to each isotope.

While using radioactive isotopes provides a rapid way to detect and quantified multiplexed samples for rapid quantitative comparison, most samples of interest also require identification by mass spectrometry at least initially. Labeling with different radioactive isotopes produces different mass of the same molecules thus results in distinct peaks in a mass spectrum. Such quality can also be exploited to make quantitative comparison especially to reconfirm previous comparison made with other methods.

### Improvements:

### Resolution:

A further improvement method for reading arrays or gels that can increase the resolution of the radiation read is the use of a magnetic field to redirect radiant electrons. Beta radiation is essentially electrons or positrons traveling at high velocity. By applying a magnetic field perpendicular to the array, any electrons or positrons not traveling in parallel with the magnetic field will experience a force and be redirected. When electrons mostly travel perpendicular to the array to strike detector or storage screen, better resolution is achieved.

Permanent magnets preferably with circular or disc shape can be placed below or above or on both sides of the exposure cassette during exposure. Magnets with other shapes can also be used as long as the magnetic line of force will pass perpendicularly through the exposure cassette. Magnets of circular disc shape are likely to have magnetic lines of force perpendicular to the circle thus when thin magnets are used this feature is helpful. In addition to permanent magnets, other electro-magnets including those with superconductor coils can also be used.

### Sensitivity and background:

An additional improvement to the labeling process is the added ability to select labeled molecules away from the rest of the samples for analysis. Both samples are first labeled with chemically similar tags containing different radioactive isotopes. These are the tags that not only can label molecules of interest with various radioactive isotopes, but also provide a means to separate and enrich the labeled molecules away from the unlabeled molecules. These types of tags can be linked to an affinity tags such as biotin, immino-biotin, 6His...so they can be quickly selected by capturing agents such as avidin, strepavidin, or nickel. These labeling tags can also be linked to beads such as agarose, sepharose®, paramagnetic beads so that labeled molecules can be separated by physical means such as sedimentation or use of a magnetic field. These labeling tags can also be immobilized by covalent linkage to a surface of a container or reaction vessel.

While affinity tags can remain with the labeled molecules of interest throughout the analysis, sometimes it is desirable to remove them. Biotin is a small affinity tags that by itself can carry ³H, ¹⁴C, or ³⁵S. Biotin can be linked to other conjugation reactive groups such as NHS to target specific molecules such as Lysine residues on proteins. A cleavable linkage between the affinity tags and the conjugation reactive groups can also be added so bulky biotin can be removed as in the case of Biotin-SS-NHS. If such a labeling agent is used then the labels need to be in the conjugation reactive group (NHS in this case), additionally, the sulfur next to NHS can also be ³⁵S labeled, The disulfide linkage can easily be cleaved by the addition of a reducing reagent such as dithiothreitol or 2-mecaptoethanol after affinity selection.

Other selection means such as beads and immobilization must have a means to release the labeled molecules of interest with a part of the label (with the radioactive isotope of choice) still attached. One such means is the disulfide SS linkage described above. Another mean is the use of a photo-cleavable linkage. An additional means is to use a chemically or enzymatically cleavable linkage. Many such linkages are available and known to those skilled in the art.

The above description provides a methodology for which a broad spectrum of particular analysis may apply. Accordingly, the following examples are provided to further enhance the users understanding of the invention but in no way are intended to limit the particular application of the method described above.

### EXAMPLES

### Example 1: Biomarker discovery and high-throughput assay development (comparative example)

Because of the limited antibodies availability, biomarkers must first be discovered and then antibodies are made against them to build useful antibody arrays for high throughput screening. Current methods use 2-D gel electrophoresis to separate proteins. This example presents an alternative to 2-D gel electrophoresis.

Samples from two sources such as drug-treated and vehicle treated cells are used to look for biomarkers. These samples are mixed together for analysis. A small aliquot is counted on scintillation counter capable of distinguishing between ³H and ¹⁴C to establish the ratio of samples mixture. The mixture is then separated by tandem chromatography. The fractions resulting from one type of chromatography are further separated by another form of chromatography. Every fraction produced is monitored for ³H/¹⁴C ratio to select for fractions with significant ratio deviation. These fractions of interest can be analyzed further by SDS-PAGE and applied to the two screen system to quantify ³H/¹⁴C ratio. The bands showing significant ratio difference are cut out for identification by mass spectrometry. During mass spectrometry analysis, the mass difference between molecules labeled with different isotopes will also become apparent. For instance, labeling with a methyl group using ¹⁴C increase the molecular mass by 2 atomic mass units (amu) while labeling with a methyl group containing all three tritium increase the molecular mass by 6 amu. The result is that a ¹⁴C-labeled molecule is 4 amu lighter than a tritium-labeled molecule for every methyl group used. This difference allows the operator to tell whether the correct proteins are being examined and also serves to reconfirm the previous quantification results.

Antibodies are made against the identified proteins or partial protein sequences resulting from mass spectrometry analyses. These antibodies are used to make antibody arrays to rapidly screen for the same biomarker changes in micro format. The array is exposed to the same mixture from original samples to validate their effectiveness in identifying the biomarkers whose changes are originally found by other methods. Once validated, the arrays are ready for high-throughput screening use.

### Example 2: Post-translational modification studies

### Protein phosphorylation study

Post-translational modifications of proteins are very important regulatory mechanism in eukaryotic organisms. One such important modification is phosphorylation: a phosphate added to an enzyme or receptor can turn it on or off depending on the type of enzyme or receptor. Thus a system that enables the comparison of protein phosphorylation with high degree of sensitivity can be a very powerful tool for research and diagnostic use. A dual labeling system using chemically similar labeling agents that comprises either ³²P or ³³P is used to determine the degree of phosphorylation in two set of cells or cells undergoing two different treatments.

Cellular drug response can be studied as followed: (1) Cells are grown equally in two culture dishes. Prior to labeling, the cells are starved of phosphate by washing and replacing growth media with phosphate-deficient media for 1 hour. (2) Labeling media are added wherein one contains ³²P phosphates while the other contains ³³P phosphates. At the same time one set is (or had been) treated with drug while the other is treated with vehicles (any solvent used to carry the drug) or another drug for efficacy comparison. (3) After a predetermined amount of treatment time, radioactive media is washed away and the cells are harvested and lysed in RIPA buffer with phosphatase inhibitors such as NaF and okadaic acid. The cell lysates are mixed together and subjected to different analyses as described below.

### Electrophoresis-

SDS-PAGE and 2-D gel electrophoresis are performed to separate the different proteins for analysis. The gels are then fixed in 10% acetic acid and dry on gel drier. Comparative example: Each dried gel is exposed to special 2-layer phosphorescent imaging storage screen comprising of one thin screen made of phosphorescent and backing material just enough so that most of the ³³P signal cannot pass through. The other screen is also made of the identical phosphorescent material but placed on much better backing plate. The first screen was designed to capture both radiation signals from ³²P and ³³P while allowing much higher proportion of radiation signal from ³²P to pass through to the second screen. Since ³³P signal is relatively weak, the first screen can be thin enough so that the signal picked up on the second screen is not significantly more diffused. Additionally, a strong magnetic field can be used to minimize such diffusion. Then the screens are read using phospho-imaging reader and the result interpreted by specialized software. Using known internal standards for calibration such as signal unique to just ³³P or ³²P, then the same signals from sample mixtures are calculated and compared for bands or spots of proteins of interest. According to the present invention, each dried gel can be exposed to a single phosphorescent imaging screen, then read, then expose to the same screen again then read again after a period of radioactive decay, then repeat if necessary. Use the reading before and after a period of radioactive decay to enable quantification of ³²P and ³³P in each band or spot and thus identify bands or spots with deviated ratio of radioactive isotopes. From these calculations, the relative amount of phosphorylation for each protein between samples is calculated. These differences can assess the relative drug effectiveness.

### Immuno-precipitation-

The mixed cell lysate is subjected to immuno-precipitation to study the phosphorylation and its effect on protein interaction. Lysate is precleared with protein G coupled to sepharose beads. Then appropriate antibody is added and incubated for 2 hours followed by the addition of protein G sepharose beads for an additional hour. Beads are collected by centrifugation, washed for at least three times to remove non-specific interaction, and then subject to SDS-PAGE analysis. The gel is fixed, dried marked with control markers, and then exposed to the two-layer phosphorescent imaging screen system designed for ³²P and ³³P. The screens are read and the bands of interest are quantified and normalized with control markers enabling quantitative comparison between the two samples (comparative example). According to the present invention, proteins co-labeled with ³H and ¹⁴C for interaction studies can also be differentiated and quantified especially after a decay period.

### Tandem/multidimensional chromatography

As described earlier, different methods of chromatography can be used to look for proteins with variation in phosphorylation when apply to this experiment. The isolated proteins can be identified by existing methods including mass spectrometry.

### Determination of the degree of protein phosphorylation in clinical samples:

A variation of the above method is used to compare the degree of phosphorylation for one or a plurality of proteins between two clinical samples. The sample are fractionated to some degree of purity suitable for the experiment before one of the sample is subjected to kinase phosphorylation using ³²P ATP while the other sample with ³³P ATP. The kinases will add phosphate groups to phosphorylation sites that do not already have phosphates. Because there are many kinases and each of them will specifically phosphorylate different sites, this method can target specific protein or family of proteins. After kinases reactions, the resulting proteins are mixed together for various types of analyses as outlined above.

### Tritium and ¹⁴C-labeling for glycosylation study (comparative example).

Other posttranslational modification such as glycosylation can also be studied using ³H and ¹⁴C substrates. The detection using a two-layer phosphorescent screen can be difficult since the first screen should be as thin as a saran wrap for ¹⁴C signal to go through and construction of such screen is so prone to damage. To solve this technical problem, the gel destined for analysis is dried onto a thin support film that would block most of the ³H signal but allow a good proportion of ¹⁴C signal to pass. The gel is then exposed to two phosphorescent screens: one on the gel side and one on the thin film backing side. A less convenient method is to expose the gel to one screen, and then expose again to a similar screen with a built-in tritium blocking screen for the same amount of time. The signals are then quantified accordingly. In addition to quantification done by phosphor-imager, the dots or bands of interest can also be cut out for scintillation counting, or mass spectrometry analysis. Scintillation counter technology is advanced enough to enable the instrument to distinguish the difference between emissions resulting from ³H versus that resulting from ¹⁴C. While the main quantification method for radioactivity presented here uses phosphoimaging screen and scintillation counting, other methods, variations and combinations of existing methods can be used and are apparent to those skilled in the art.

### Example 3: Genomic/RNA expression studies

For tissue samples where RNA is readily extracted, the RNA can be labeled with either ³²P or ³³P at their end terminals and then applied to a complementary DNA array. The array is then exposed to the two-screen phosphorescent imaging system so signals specific to either ³²P or ³³P could be quantified and compared after normalized with internal controls' signals (comparative example). According to the present invention, total signals can be quantified before and after a few days and used to calculate signal unique to ³²P or ³³P. The half-life of ³²P is shorter than that of ³³P (14.3 days compared to 25.3 days) thus makes the calculation possible. For higher sensitivity in smaller tissue sample, a Reverse Transcriptase reaction can be performed with either ³²P or ³³P labeled poly dT or labeled nucleotides. The poly dT is hybridized to the poly A tail of mRNA and reverse transcriptase synthesizes the rest of the complementary sequence to form a single stranded cDNA. The RNA is then digested away by RNAse and the cDNA is applied to an array for analysis.

In addition to comparing mRNA expression for the same genes between the two tissue samples, these procedures can also compare the expression level of different genes in the same tissue because each RNA molecule is labeled with the same amount of radioactivity (except when cDNA is synthesized with labeled nucleotides. For further improvement in sensitivity, radioactive nucleotides i.e. [³²P/³³P]-dATPs are used for the synthesis step so more labels are incorporated into each molecule. For this method, ³⁵S nucleotides can also be used in place of ³³P nucleotides. When using difference half-life to differentially quantify the isotopes, ³⁵S is also superior with half-life of 87.2 days compared to³³P's half-life of 25.3 days while comparing with ³²P's half-life of 14.3 days.

Other uses for the present invention are not discussed thoroughly in this disclosure; however, are still encompassed by the present invention. For example, the disclosed methods may be, of course, utilized in proteomics studies, genomics studies, metabolomics, glycomics studies, and in vitro diagnostic applications. In addition to using the invention to make abundance comparison of similar molecules or molecular characteristics, using known amount to quantify an unknown amount of similar molecules is also possible. Specific details with regard to these particular uses for the present invention are known and understood by one skilled in the art.

It should be clear that the methods disclosed in this invention may be coupled with various other studies, or used with many other means of analysis. While the description emphasizes studies of proteins, DNA and RNA; other molecules can be studied with variations of this method.

Having disclosed my invention in such terms as to enable those skilled in the art to understand and practice it, and, having identified the presently preferred embodiments thereof, I CLAIM:

## Claims

1. A method for comparing samples comprising the steps of:
a) providing two samples for comparative analysis;
b) labeling molecules in a first sample by covalent linkage with a first radioactive isotope;
c) labeling molecules in a second sample by covalent linkage with a second radioactive isotope which has a different half-life compared to the half-life of said first radioactive isotope;
d) mixing said first sample and said second sample into a homogeneous mixture;
e) subjecting said homogeneous mixture to any means of separation that can separate the molecules into fractions or bands or dots;
f) quantifying a total amount of radiation from each fraction or band or dot;
g) quantifying a total amount of post-decay radiation from said each fraction or band or dot after a period of radioactive decay;
h) examining every fraction's or band's or dot's total radiation and total post-decay radiation whereby any fraction or band or dot with a deviated ratio of total radiation to total post-decay radiation can be identified.

2. The method of claim 1 further comprises a step of isolating a molecule that causes said deviated ratio of total radiation to total post-decay radiation from one of the fraction or band or dot with deviated ratio of total radiation to total post-decay radiation.

3. The method of claim 2 further comprises a step of identifying said molecule that causes said deviated ratio of total radiation to total post-decay radiation by mass spectrometry.

4. The method of claim 1 further comprises a step of applying a magnetic field with the magnetic lines of force perpendicular to the plane of a gel or array to improve beta radiation resolution when said gel or array is used as said means of separation.

5. A method for isolating a differentially abundant molecule between two samples comprising the steps of:
a) providing two samples for analysis;
b) labeling molecules in a first sample by covalent linkage with a first radioactive isotope;
c) labeling molecules in a second sample by covalent linkage with a second radioactive isotope which has a different half-life compared to the half-life of said first radioactive isotope;
d) mixing said first sample and said second sample into a homogeneous mixture;
e) subjecting said homogeneous mixture to any means of separation that can separate molecules into fractions or bands or dots;
f) using the difference in half-life between said first radioactive isotope and said second radioactive isotope to identify a fraction or a band or a dot with deviated radioactive isotopes' ratio; and,
g) isolating said differentially abundant molecule from said fraction or said band or said dot with deviated radioactive isotopes' ratio wherein said differentially abundant molecule is responsible for causing said deviated radioactive isotopes' ratio.

6. The method of claim 5 further comprises a step of identifying said differentially abundant molecule by mass spectrometry.

7. A method for quantifying a molecule in a sample comprising the steps of:
a) providing a first sample with unknown amount of said molecule for analysis;
b) providing a second sample with known amount of said molecule for analysis;
c) labeling molecules in a first sample by covalent linkage with a first radioactive isotope;
d) labeling molecules in a second sample by covalent linkage with a second radioactive isotope which has a different half-life compared to the half-life of said first radioactive isotope;
e) mixing said first sample and said second sample into a homogeneous mixture;
f) subjecting said homogeneous mixture to any means of separation that can separate the molecules into fractions or bands or dots;
g) identify a fraction or a band or a dot that contains said molecule;
h) quantifying a total amount of radiation from said fraction or said band or said dot;
i) quantifying a total amount of post-decay radiation from said fraction or said band or said dot after a period of radioactive decay;
j) using the ratio of said total amount of radiation to said total amount of post-decay radiation to calculate said unknown amount of said molecule in said first sample according to said known amount of said molecule in said second sample.

## Patentansprüche

1. Verfahren zum Vergleichen von Proben, umfassend die Schritte:
a) das Bereitstellen von zwei Proben zur vergleichenden Analyse;
b) das Markieren von Molekülen in einer ersten Probe durch kovalente Verknüpfung mit einem ersten radioaktiven Isotop;
c) das Markieren von Molekülen in einer zweiten Probe durch kovalente Verknüpfung mit einem zweiten radioaktiven Isotop, das eine im Vergleich zu der Halbwertszeit des ersten radioaktiven Isotops unterschiedliche Halbwertszeit hat;
d) das Vermischen der ersten Probe und der zweiten Probe zu einem homogenen Gemisch;
e) das Unterwerfen des homogenen Gemisches einem beliebigen Mittel zur Trennung, welches die Moleküle in Fraktionen, Banden oder Dots trennen kann;
f) das Quantifizieren einer Gesamtmenge an Strahlung von jeder Fraktion oder jeder Bande oder jedem Dot;
g) das Quantifizieren einer Gesamtmenge an Strahlung nach dem Zerfall von besagter jeder Fraktion oder jeder Bande oder jedem Dot im Anschluss an einen Zeitraum des radioaktiven Zerfalls;
h) das Untersuchen der Gesamtstrahlung und der Gesamtstrahlung nach dem Zerfall einer jeden Fraktion oder einer jeden Bande oder eines jeden Dots, wodurch eine beliebige Fraktion oder eine beliebige Bande oder eine beliebiger Dot mit einem abweichenden Verhältnis der Gesamtstrahlung zu der Gesamtstrahlung nach dem Zerfall identifiziert werden kann.

2. Verfahren nach Anspruch 1, des Weiteren umfassend einen Schritt des Isolierens eines Moleküls, das ursächlich für das abweichende Verhältnis der Gesamtstrahlung zu der Gesamtstrahlung nach dem Zerfall ist, aus einer der Fraktionen oder Banden oder aus einem der Dots mit einem abweichenden Verhältnis der Gesamtstrahlung zu der Gesamtstrahlung nach dem Zerfall.

3. Verfahren nach Anspruch 2, des Weiteren umfassend einen Schritt des Identifizierens des besagten Moleküls, das ursächlich für das abweichende Verhältnis der Gesamtstrahlung zu der Gesamtstrahlung nach dem Zerfall ist, mittels Massenspektrometrie.

4. Verfahren nach Anspruch 1, des Weiteren umfassend einen Schritt des Anwendens eines Magnetfelds, in dem die magnetischen Kraftlinien senkrecht zu der Ebene eines Gels oder Arrays verlaufen, um die Auflösung von Betastrahlung zu verbessern, wenn das Gel oder der Array als das besagte Mittel zur Trennung verwendet wird.

5. Verfahren zum Isolieren eines Moleküls mit einer unterschiedlichen Häufigkeit in zwei Proben, umfassend die folgenden Schritte:
a) das Bereitstellen von zwei Proben zur Analyse;
b) das Markieren von Molekülen in einer ersten Probe durch kovalente Verknüpfung mit einem ersten radioaktiven Isotop;
c) das Markieren von Molekülen in einer zweiten Probe durch kovalente Verknüpfung mit einem zweiten radioaktiven Isotop, das eine im Vergleich zu der Halbwertszeit des ersten radioaktiven Isotops unterschiedliche Halbwertszeit hat;
d) das Vermischen der ersten Probe und der zweiten Probe zu einem homogenen Gemisch;
e) das Unterwerfen des homogenen Gemisches einem beliebigen Mittel zur Trennung, welches die Moleküle in Fraktionen, Banden oder Dots trennen kann;
f) das Verwenden des Unterschieds zwischen den Halbwertszeiten des besagten ersten radioaktiven Isotops und des besagten zweiten radioaktiven Isotops zum Identifizieren einer Fraktion oder einer Bande oder eines Dots mit einem abweichenden Verhältnis der radioaktiven Isotopen; und
g) das Isolieren des besagten Moleküls mit der unterschiedlichen Häufigkeit aus der besagten Fraktion oder der besagten Bande oder aus dem besagten Dot mit einem abweichenden Verhältnis der radioaktiven Isotopen, wobei das Molekül mit der unterschiedlichen Häufigkeit ursächlich für das besagte abweichende Verhältnis der radioaktiven Isotopen ist.

6. Verfahren nach Anspruch 5, weiters umfassend einen Schritt des Identifizierens des besagten Moleküls mit der unterschiedlichen Häufigkeit mittels Massenspektrometrie.

7. Verfahren zum Quantifizieren eines Moleküls in einer Probe, umfassend die folgenden Schritte:
a) das Bereitstellen einer ersten Probe mit einer nicht bekannte Menge des besagten Moleküls zur Analyse;
b) das Bereitstellen einer zweiten Probe mit einer bekannte Menge des besagten Moleküls zur Analyse;
c) das Markieren von Molekülen in einer ersten Probe durch kovalente Verknüpfung mit einem ersten radioaktiven Isotop;
d) das Markieren von Molekülen in einer zweiten Probe durch kovalente Verknüpfung mit einem zweiten radioaktiven Isotop, das eine im Vergleich zu der Halbwertszeit des besagten ersten radioaktiven Isotops unterschiedliche Halbwertszeit hat;
e) das Vermischen der ersten Probe und der zweiten Probe zu einem homogenen Gemisch;
f) das Unterwerfen des homogenen Gemisches einem beliebigen Mittel zur Trennung, welches die Moleküle in Fraktionen, Banden oder Dots trennen kann;
g) das Identifizieren einer Fraktion oder einer Bande oder eines Dots, die bzw. der das Molekül enthält;
h) das Quantifizieren einer Gesamtmenge an Strahlung von besagten Fraktion oder Bande oder Dot;
i) das Quantifizieren einer Gesamtmenge an Strahlung nach dem Zerfall der besagten Fraktion oder der Bande oder des Dots im Anschluss an einen Zeitraum des radioaktiven Zerfalls; und
j) das Verwenden des Verhältnisses der besagten Gesamtmenge an Strahlung zu der besagten Gesamtmenge an Strahlung nach dem Zerfall zum Berechnen der besagten nicht bekannten Menge des besagten Moleküls in der besagten ersten Probe gemäß der besagten bekannten Menge des besagten Moleküls in der besagten zweiten Probe.

## Revendications

1. Procédé de comparaison d'échantillons, comprenant les étapes suivantes :
a) la fourniture de deux échantillons à des fins d'analyse comparative ;
b) le marquage des molécules d'un premier échantillon par liaison covalente avec un premier isotope radioactif ;
c) le marquage des molécules d'un second échantillon par liaison covalente avec un second isotope radioactif qui a une demi-vie différente de la demi-vie dudit premier isotope radioactif ;
d) le mélange dudit premier échantillon et dudit second échantillon pour obtenir un mélange homogène ;
e) la soumission dudit mélange homogène à de quelconques moyens de séparation aptes à séparer les molécules en fractions ou en bandes ou en points ;
f) la quantification d'une quantité totale de rayonnement émise par chaque fraction ou bande ou point ;
g) la quantification d'une quantité totale de rayonnement après désintégration émise par chacune desdites fractions ou bandes ou chacun desdits points après une période de désintégration radioactive ;
h) l'examen du rayonnement total et du rayonnement total après désintégration émis par chaque fraction ou bande ou point afin d'identifier une quelconque fraction ou bande ou un quelconque point présentant un écart du rapport entre le rayonnement total et le rayonnement total après désintégration.

2. Procédé selon la revendication 1, comprenant en outre une étape d'isolement d'une molécule qui provoque ledit écart du rapport entre le rayonnement total et le rayonnement total après désintégration, de l'un de la fraction ou de la bande ou du point présentant un écart du rapport entre le rayonnement total et le rayonnement total après désintégration.

3. Procédé selon la revendication 2, comprenant en outre une étape d'identification de ladite molécule qui provoque ledit écart du rapport entre le rayonnement total et le rayonnement total après désintégration par spectrométrie de masse.

4. Procédé selon la revendication 1, comprenant en outre une étape d'application d'un champ magnétique dont les lignes de force magnétique sont perpendiculaires au plan d'un gel ou d'un réseau, pour améliorer la résolution du rayonnement bêta lorsque ledit gel ou réseau est utilisé en tant que lesdits moyens de séparation.

5. Procédé d'isolement d'une molécule présente en différentes quantités entre deux échantillons, comprenant les étapes suivantes :
a) la fourniture de deux échantillons à des fins d'analyse ;
b) le marquage des molécules d'un premier échantillon par liaison covalente avec un premier isotope radioactif ;
c) le marquage des molécules d'un second échantillon par liaison covalente avec un second isotope radioactif qui a une demi-vie différente de la demi-vie dudit premier isotope radioactif ;
d) le mélange dudit premier échantillon et dudit second échantillon pour obtenir un mélange homogène ;
e) la soumission dudit mélange homogène à de quelconques moyens de séparation aptes à séparer les molécules en fractions ou en bandes ou en points ;
f) l'utilisation de la différence de demi-vie entre ledit premier isotope radioactif et ledit second isotope radioactif pour identifier une fraction ou une bande ou un point présentant un écart du rapport des isotopes radioactifs ; et
g) l'isolement de ladite molécule présente en différentes quantités de ladite fraction ou de ladite bande ou dudit point présentant un écart du rapport des isotopes radioactifs dans lequel ladite molécule présente en différentes quantités est responsable dudit écart du rapport des isotopes radioactifs.

6. Procédé selon la revendication 5, comprenant en outre une étape d'identification de ladite molécule présente en différentes quantités par spectrométrie de masse.

7. Procédé de quantification d'une molécule dans un échantillon, comprenant les étapes suivantes :
a) la fourniture d'un premier échantillon contenant une quantité inconnue de ladite molécule à des fins d'analyse ;
b) la fourniture d'un second échantillon contenant une quantité connue de ladite molécule à des fins d'analyse ;
c) le marquage des molécules d'un premier échantillon par liaison covalente avec un premier isotope radioactif ;
d) le marquage des molécules d'un second échantillon par liaison covalente avec un second isotope radioactif qui a une demi-vie différente de la demi-vie dudit premier isotope radioactif ;
e) le mélange dudit premier échantillon et dudit second échantillon pour obtenir un mélange homogène ;
f) la soumission dudit mélange homogène à de quelconques moyens de séparation aptes à séparer les molécules en fractions ou en bandes ou en points ;
g) l'identification d'une fraction ou d'une bande ou d'un point qui contient ladite molécule ;
h) la quantification d'une quantité totale de rayonnement émise par ladite fraction ou ladite bande ou ledit point ;
i) la quantification d'une quantité totale de rayonnement après désintégration émise par ladite fraction ou ladite bande ou ledit point après une période de désintégration radioactive ;
j) l'utilisation du rapport entre ladite quantité totale de rayonnement et ladite quantité totale de rayonnement après désintégration pour calculer ladite quantité inconnue de ladite molécule dans ledit premier échantillon en fonction de ladite quantité connue de ladite molécule dans ledit second échantillon.
